# EUROPEAN PATENT APPLICATION

(11) **EP 4 834 340 A1**
(43) Date of publication of application: **04.11.2026**
(21) Application number: 24913437.0
(22) Date of filing: 27.12.2024
(51) Int. Cl.: A61K 31/351, A61K 38/26, A61K 9/00, A61P 3/04

(54) **ANTI-OBESITY COMBINATION THERAPY OF ENAVOGLIFLOZIN AND LOW-DOSE LIRAGLUTIDE**

(30) Priority: 28.12.2023 KR 20230195466
(71) Applicant: Daewoong Pharmaceutical Co., Ltd., Hwaseong-si, Gyenoggi-do 18623 (KR)
(72) Inventor: IM, Byeong Nam, Yongin-si Gyeonggi-do 17028 (KR); BAE, Byoung Chan, Yongin-si Gyeonggi-do 17028 (KR); JEONG, Hyeon Myeong, Yongin-si Gyeonggi-do 17028 (KR); PARK, Joon Seok, Yongin-si Gyeonggi-do 17028 (KR); CHOI, Ji-Soo, Yongin-si Gyeonggi-do 17028 (KR); JI, Hye Young, Yongin-si Gyeonggi-do 17028 (KR); LIM, Hyun-Woo, Yongin-si Gyeonggi-do 17028 (KR)
(74) Representative: Plasseraud IP
(86) International application number: PCT/KR2024/021202
(87) International publication number: WO 2025/143830

(57) **Abstract**

The present invention provides an obesity treatment regimen with reduced side effects through low-dose combined administration of enavogliflozin and liraglutide. A combination therapy of enavogliflozin and low-dose liraglutide provides a synergistic effect in obesity treatment compared to single administration of each drug. In addition, compared to single administration of liraglutide, an administration dose of liraglutide may be reduced, and thus a direct cause of a side effect related to a gastrointestinal system and a cardiovascular system of liraglutide may be reduced. In addition, through combination of enavogliflozin, it has an advantage of additionally providing a metabolic disease and cardiovascular disease improvement effect related to blood glucose, blood lipid, blood pressure, fatty liver, and the like.

## Description

### [Technical Field]

The present invention relates to an anti-obesity combination therapy of enavogliflozin and low-dose liraglutide.

### [Background Art]

A GLP-1 receptor agonist (GLP-1 Receptor Agonist, hereinafter also referred to as GLP-1RA) is a drug that activates GLP-1 receptors to reduce blood glucose and energy absorption. The GLP-1 receptor agonist mimics the action of GLP-1, an endogenous incretin hormone secreted from the intestine after a meal. GLP-1 receptor agonists inhibit glucagon secretion, stimulate insulin production to reduce hyperglycemia in patients with type 2 diabetes, and are effective in treating obesity through weight loss by reducing food intake. The inhibition of glucagon secretion by GLP-1 receptor agonists occurs in proportion to glucose concentration. That is, it has the advantage of not causing serious problems in a hypoglycemic situation because glucagon secretion is inhibited at high blood glucose and glucagon secretion is not inhibited at low blood glucose. In the case of liraglutide, a GLP-1RA drug well known under the trade name Saxenda, it was developed as a therapeutic agent for diabetes in the early stage of development, but it is known to be effective against obesity when administered at a high dose, and is currently widely used for the treatment of obesity. However, the prescribed dose of liraglutide for obesity reaches 3 to 5 times the prescribed dose for diabetes, and unlike when administering the prescribed dose for diabetes, it is known that when administering the prescribed dose for obesity, there are a number of side effects, including gastrointestinal side effects such as nausea and diarrhea, and cardiovascular side effects such as an increased heart rate due to the high-dose prescription.

Meanwhile, SGLT-2 inhibitors are drugs having a mechanism of weight reduction and blood pressure reduction effects through osmotic diuresis and caloric loss caused by glucose excretion. Enavogliflozin, an SGLT-2 inhibitor developed by the present applicant, is a drug recently approved for marketing as an oral therapeutic agent for diabetes. According to a prior study of the present applicant, it was confirmed that enavogliflozin is excellent in obesity treatment effect based on the ability to inhibit glucose reabsorption.

Accordingly, the present inventors conducted research under the hypothesis that the combination administration of enavogliflozin and low-dose liraglutide would cause synergy in the treatment of obesity, thereby improving the anti-obesity effect while reducing side effects compared to conventional therapeutic agents. As a result, they confirmed that the combination administration of enavogliflozin and low-dose liraglutide reduces the side effects of liraglutide and exhibits a synergistic effect in weight reduction compared to the single administration of the two drugs, thereby completing the present invention.

### [Disclosure]

### [Technical Problem]

An object of the present invention is to provide a novel anti-obesity therapy with improved anti-obesity effects while reducing side effects due to high-dose administration of liraglutide.

### [Technical Solution]

The present invention provides a pharmaceutical composition for use in anti-obesity comprising enavogliflozin as an active ingredient, characterized by combination with a low dose of liraglutide.

In another embodiment, the present invention provides a pharmaceutical composition for use in anti-obesity comprising a low dose of liraglutide as an active ingredient, characterized by combination with enavogliflozin.

In the pharmaceutical composition of the present invention, enavogliflozin or liraglutide includes all cases where it exists in the form of a pharmaceutically acceptable salt, a pharmaceutically acceptable amide, or a pharmaceutically acceptable ester thereof.

Enavogliflozin is a drug currently approved as a therapeutic agent for diabetes and is sold under the product name Envlo Tablet. The dose of enavogliflozin as a therapeutic agent for diabetes was approved as 0.3 mg per day, but the dose of enavogliflozin as a therapeutic agent for obesity has not been determined. The present invention relates to an anti-obesity treatment use of enavogliflozin for combination administration with a low dose of liraglutide, and based on the following examples, the dose of enavogliflozin for combination administration together with a low dose of liraglutide may be 0.3 to 1.5 mg/day.

Here, the low-dose of liraglutide refers to a low level of dose compared to the dose of liraglutide currently used for the treatment of obesity. Liraglutide is currently approved for two uses, diabetes and obesity. While the dose for the treatment of diabetes is 0.6 to 1.8 mg daily, the dose for the treatment of obesity is 3.0 mg daily, and the approved dose for obesity treatment is much higher than the approved dose for diabetes treatment. The low-dose of liraglutide intended in the present invention means a dose of less than 3.0 mg daily, which is the current approved dose for obesity treatment, and although not limited thereto, the dose of liraglutide may be 1.2 to 2.4 mg/day. That is, when enavogliflozin and a low dose of liraglutide are administered in combination, in one embodiment, the dose of enavogliflozin may be 0.3 to 1.5 mg/day, and the dose of liraglutide may be 1.2 to 2.4 mg/day. In another embodiment, the dose of enavogliflozin may be 0.6 to 1.2 mg/day, and the dose of liraglutide may be 1.5 to 2 mg/day.

The term "combination" as used in the present invention means that enavogliflozin and liraglutide are administered together to a subject in need thereof. There is no temporal restriction regarding being administered together, and it includes all of being administered individually, simultaneously, or sequentially. In addition, "combination" includes both the case where enavogliflozin and liraglutide are individually administered as separate formulations and the case where they are administered as a combination formulation comprising enavogliflozin and liraglutide.

In combining the two drugs, the usage is not particularly limited. Enavogliflozin is a drug initially developed as an oral agent, but its parenteral administration is not limited. For the treatment of obesity, liraglutide is currently administered parenterally. Therefore, for example, it is possible to administer enavogliflozin orally or parenterally, while liraglutide is administered parenterally. A specific example of parenteral administration suitable for the present invention may be subcutaneous or intramuscular administration. Liraglutide for anti-obesity is currently administered once a day through subcutaneous injection.

Although not limited thereto, in one embodiment of the present invention, enavogliflozin may be administered orally and liraglutide may be administered through subcutaneous injection.

In another embodiment, enavogliflozin and liraglutide may be administered through subcutaneous injection.

In the following examples, it was confirmed that an increased anti-obesity effect can be exhibited even with a low dose of liraglutide both in the case where enavogliflozin was administered orally and liraglutide was administered in combination through subcutaneous injection, and in the case where a depot composition was prepared as a combination formulation comprising enavogliflozin and liraglutide and these drugs were administered in combination.

That is, the difference in the route of administration of each drug does not affect the achievement of the increased anti-obesity effect according to the combination of enavogliflozin and liraglutide.

### [Advantageous Effects]

The combination therapy of enavogliflozin and low-dose liraglutide provides a synergistic effect in the treatment of obesity compared to the single administration of each drug. In addition, compared to the single administration of liraglutide, the administered dose of liraglutide can be reduced, thereby reducing the direct cause of side effects related to the gastrointestinal system and cardiovascular system of liraglutide. In addition, through the combination of enavogliflozin, it has the advantage of additionally providing metabolic disease and cardiovascular disease improvement effects related to blood glucose, blood lipids, blood pressure, fatty liver, and the like.

### [Description of Drawings]

Fig. 1 is a graph showing the body weight reduction effect according to the combination administration of enavogliflozin and liraglutide in a high-fat diet-induced obesity mouse model.
Fig. 2 is a graph showing the change in body weight (%) according to the combination administration of enavogliflozin and liraglutide in a high-fat diet-induced obesity mouse model.
Fig. 3 shows the results of evaluating the difference in body weight reduction efficacy by the combination administration of enavogliflozin and liraglutide according to the degree of obesity in a high-fat diet-induced obesity mouse model.
Fig. 4 shows the body composition analysis results according to the combination administration of enavogliflozin and liraglutide in a high-fat diet-induced obesity mouse model.
Fig. 5 shows the results of an oral glucose tolerance test performed after the combination administration of enavogliflozin and liraglutide in a high-fat diet-induced obesity mouse model.
Fig. 6 is a graph showing the body weight reduction rate in an obesity-induced rat model according to the administration of the depot composition according to the present invention.
Fig. 7 is a photograph showing that the fat ratio in tissues is reduced as a body composition analysis result of an obesity-induced rat model according to the administration of the depot composition of the present invention.
Fig. 8 shows the PK analysis results of enavogliflozin according to the administration of each test substance in the test groups of G1 to G9.
Fig. 9 shows the PK analysis results of liraglutide according to the administration of each test substance in the test groups of G1 to G9.

### [Mode for Invention]

The advantages and features of the present invention, and methods for achieving them, will become clear with reference to the examples described in detail below. However, the present invention is not limited to the examples disclosed below, but will be implemented in various different forms, and these examples are merely provided to complete the disclosure of the present invention and to fully inform those of ordinary skill in the art of the scope of the invention to which the present invention belongs, and the present invention is only defined by the scope of the claims.

In the tables and drawings of the following examples, for convenience, liraglutide is also expressed by the abbreviation LIRA, and enavogliflozin is also expressed by the abbreviation ENAVO.

### [Experimental Example 1] Evaluation of anti-obesity efficacy according to combination administration of enavogliflozin and liraglutide in an obesity animal model

In order to confirm whether the combination administration of enavogliflozin and liraglutide exhibits a synergistic effect on anti-obesity efficacy, the following experiment was performed.

6-week-old male C57BL/6J mice were acclimated for 1 week, and a high-fat diet was fed for 15 weeks to prepare a diet-induced obesity mouse model (C57BL/6J 6w, male + HFD 15w, n=11~12). The diet-induced obesity mouse model was divided into the following groups, and over 4 weeks, once a day, enavogliflozin (3 mg/kg or 10 mg/kg) was orally administered and liraglutide (10 nmol/kg) was subcutaneously administered, and body weight was measured once every 4 days.

**[Table 1]**

| Group | Group Name | Number of Animals (n) | Administered Substance |
|---|---|---|---|
| G1 | Lean | 6 | Saline + Vehicle |
| G2 | Vehicle | 11 | Saline + Vehicle |
| G3 | Liraglutide | 11 | Liraglutide 10nmol/kg + Vehicle |
| G4 | Enavogliflozin 3mg/kg | 12 | Saline+Enavogliflozin 3mg/kg |
| G5 | Enavogliflozin 10mg/kg | 12 | Saline+Enavogliflozin 10mg/kg |
| G6 | Liraglutide + Enavogliflozin 3mg/kg | 12 | Liraglutide 10nmol/kg + Enavogliflozin 3mg/kg |
| G7 | Liraglutide + Enavogliflozin 10mg/kg | 12 | Liraglutide 10nmol/kg + Enavogliflozin 10mg/kg |

After 4 weeks, non-fasting blood glucose, HbA1c, and oral glucose tolerance tests were performed, while body fat mass, organ weight, and blood biochemical tests were performed.

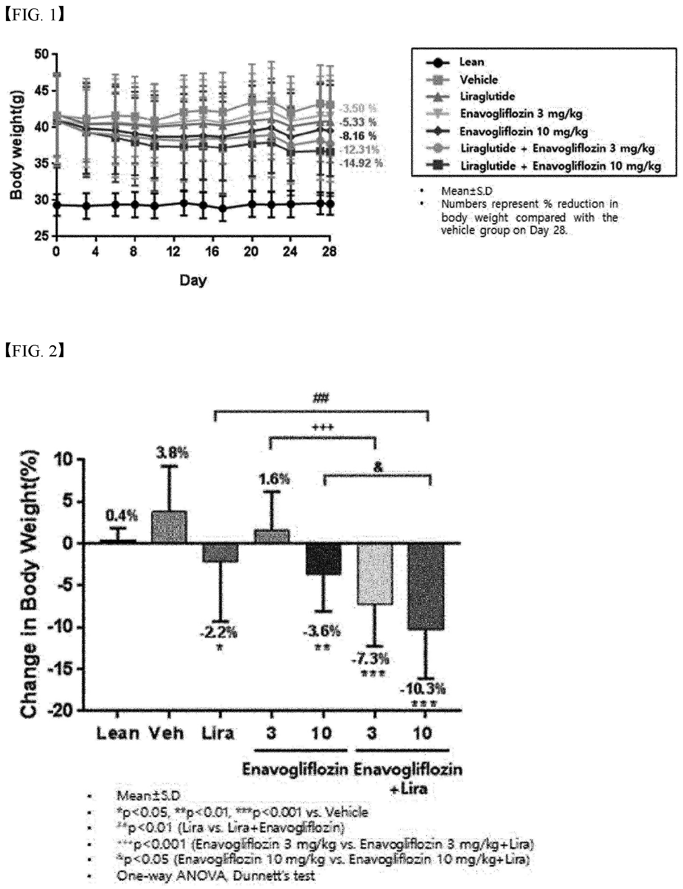

Fig. 1 is a graph showing the body weight reduction effect according to the combination administration of enavogliflozin and liraglutide in a high-fat diet-induced obesity mouse model.

As can be seen in Fig. 1, in the body weight measured on the 28th day of the experiment, compared to G2, which is the obesity mouse administered with vehicle, G3 administered with liraglutide 10 nmol/kg alone exhibited 5.33%, G4 administered with enavogliflozin 3 mg/kg exhibited 3.5%, G5 administered with enavogliflozin 10 mg/kg exhibited 8.16%, G6 administered with liraglutide 10 nmol/kg + enavogliflozin 3 mg/kg exhibited 12.31%, and G7 administered with liraglutide 10 nmol/kg + enavogliflozin 10 mg/kg exhibited 14.92% of body weight reduction. It was confirmed that the combination administration of liraglutide and enavogliflozin exhibits an increased effect in body weight reduction compared to the single administration of each drug.

Fig. 2 is a graph showing the change in body weight (%) according to the combination administration of enavogliflozin and liraglutide in a high-fat diet-induced obesity mouse model.

Looking at the change in body weight (%) of the mice of G1 to G7 in Fig. 2, similarly to Fig. 1, it was confirmed that the change in body weight of G6 administered with liraglutide 10 nmol/kg + enavogliflozin 3 mg/kg and G7 administered with liraglutide 10 nmol/kg + enavogliflozin 10 mg/kg was significantly higher than the single administration of liraglutide.

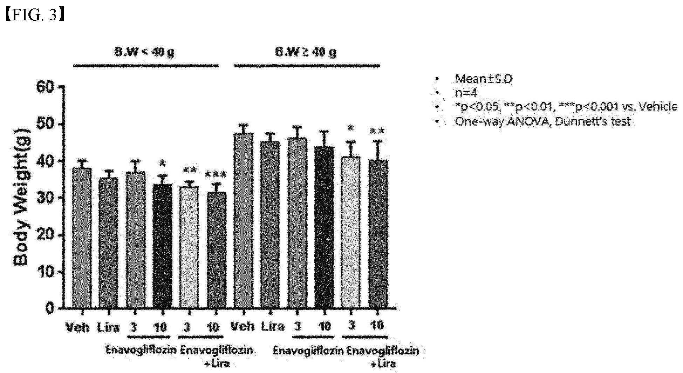

Fig. 3 shows the results of evaluating the difference in body weight reduction efficacy by the combination administration of enavogliflozin and liraglutide according to the degree of obesity in a high-fat diet-induced obesity mouse model. As a result of dividing into a group with a body weight of less than 40 g and a group with a body weight of 40 g or more and evaluating the body weight reduction efficacy compared to the initial body weight (Day 0), as can be seen in Fig. 3, it was confirmed that the anti-obesity effect of G6 administered with liraglutide 10 nmol/kg + enavogliflozin 3 mg/kg and G7 administered with liraglutide 10 nmol/kg + enavogliflozin 10 mg/kg was significant. In particular, the body weight reduction efficacy in the subgroup with a relatively low degree of obesity (B.W < 40 g) was found to be prominent.

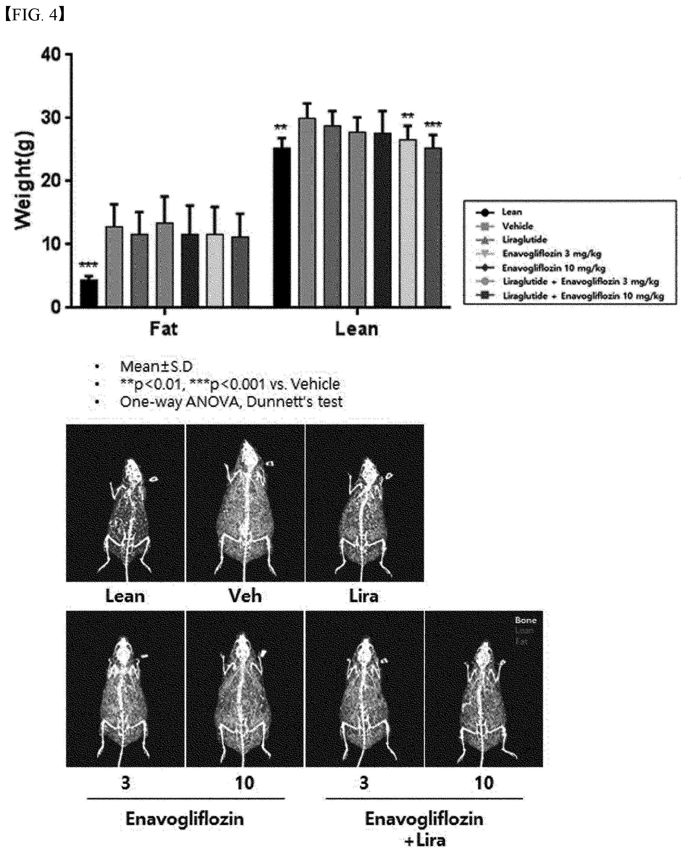

Fig. 4 shows the body composition analysis results according to the combination administration of enavogliflozin and liraglutide in a high-fat diet-induced obesity mouse model. As a result of observing the body composition after combination administration for 4 weeks, the body fat mass of the vehicle-administered group was significantly increased compared to the Lean group, and as can be seen in Fig. 4, a tendency of fat reduction appeared in all administered groups except for G4 administered with enavogliflozin 3 mg/kg, and it was confirmed that the Lean tissue was significantly reduced in the combination administration groups (liraglutide 10 nmol/kg + enavogliflozin 3 mg/kg, liraglutide 10 nmol/kg + enavogliflozin 10 mg/kg).

As a result of measuring metabolic parameters, as can be seen in Table 2, the enavogliflozin single administration group increased water intake and food intake compared to the vehicle group in a dose-dependent manner, and the liraglutide administration group showed a tendency to decrease, and when enavogliflozin and liraglutide were administered in combination, water intake and food intake showed a tendency to decrease compared to the enavogliflozin single administration group. Upon administration of enavogliflozin, food intake increases due to a compensatory mentality caused by glucose loss, but when liraglutide is administered in combination, appetite is suppressed, which is judged to exhibit an increased effect on body weight reduction in the combination administration group.

**[Table 2]**

| Parameter | Lean | Veh | Lira | Enavogliflozin 3 mg/kg | Enavogliflozin 10 mg/kg | Enavogliflozin Enavogliflozin 3 mg/kg 10 mg/kg | |
|---|---|---|---|---|---|---|---|
| | | | | | | + Lira | |
| Non-fasting Blood glucose (mg/dL) | 153.67±11.11** | 188.55±23.56 | 181.91±19.87 | 145.75±20.67*** | 132.58±18.06*** | 129.92±10.52*** | 115.75±15.06*** |
| HbAlc (%) | 4.20±0.28 | 4.49±0.38 | 4.31±0.23 | 4.35±0.22 | 4.07±0.34** | 4.23±0.21 | 4.23±0.26 |
| Water intake (mL/day/head) | 4.32 | 2.77 | 2.64 | 6.00 | 7.75 | 5.90 | 4.83 |
| Food intake (g/day/head) | 3.59 | 2.81 | 2.40 | 3.71 | 3.82 | 3.05 | 3.01 |

| | | | | | | | |
|---|---|---|---|---|---|---|---|
| • Mean ± SD • *P < 0.005, **P < 0.01, ***P < 0.001 vs Vehicle • One-way ANOVA Dunnett's text | | | | | | | |

In addition, the non-fasting blood glucose of the vehicle group was significantly increased compared to the Lean group, and the enavogliflozin 3 and 10 mg/kg single administration groups, the liraglutide 10 nmol/kg + enavogliflozin 3 mg/kg and liraglutide 10 nmol/kg + enavogliflozin 10 mg/kg administration groups showed significantly reduced blood glucose levels compared to the vehicle group. The HbA1c of all administered groups except for enavogliflozin 10 mg/kg was not significant compared to the vehicle group.

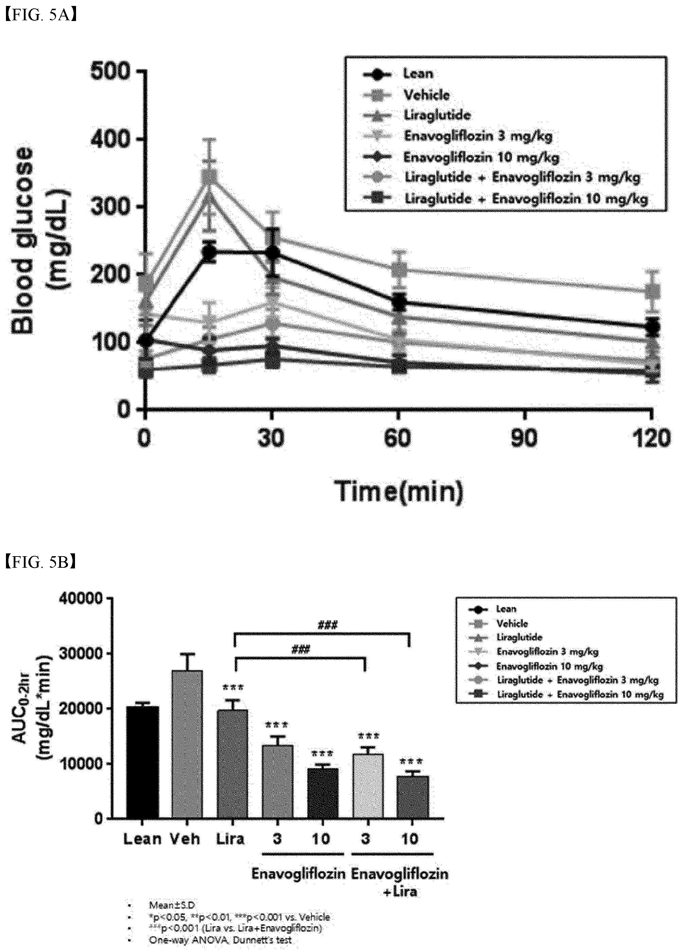

Fig. 5 shows the results of an oral glucose tolerance test performed after the combination administration of enavogliflozin and liraglutide in a high-fat diet-induced obesity mouse model. As a result of performing the oral glucose tolerance test at the 3rd week of combination administration, as can be seen in Fig. 5A, significantly lower blood glucose compared to the vehicle-administered group was observed in all enavogliflozin-administered groups at 15, 30, 60, and 120 minutes after glucose administration. Referring to Fig. 5B, as a result of comparing the AUC (Area under the curve) after glucose administration, glucose tolerance was significantly and greatly improved in all enavogliflozin-administered groups in a dose-dependent manner. Glucose tolerance was further enhanced significantly in the combination administration groups (liraglutide 10 nmol/kg + enavogliflozin 3, 10 mg/kg) compared to the liraglutide single administration group.

In addition, as a result of the blood biochemical test, as can be seen in Table 3, the enavogliflozin 10 mg/kg administration group and the liraglutide single administration group significantly reduced cholesterol (CHO) compared to the vehicle group, but a significant reduction effect was not observed in the combination administration. The enavogliflozin and liraglutide administration groups showed a tendency to decrease ALT, but significance was not observed. In AST, the enavogliflozin administration groups (single, combination) showed a tendency to increase, but it was not significant. Triglyceride (TG) was significantly increased compared to the vehicle group in the enavogliflozin 10 mg/kg administration group and the enavogliflozin 10 mg/kg + liraglutide 10 nmol/kg administration group.

**[Table 3]**

| Blood chemistry (Fasting) | Lean | Veh | Lira | Enavogliflozin 3 mg/kg | Enavogliflozin 10 mg/kg | Enavogliflozin 3 mg/kg | Enavogliflozin 10 mg/kg |
|---|---|---|---|---|---|---|---|
| | | | | | | + Li ra | |
| ALT (U/L) | 21.72±3.08* | 62.14±57.54 | 32.62±18.46 | 37.02±21.28 | 40.70±25.20 | 39.00±17.85 | 31.57±18.28 |
| AST (U/L) | 50.71±11.15 | 85.40±35.95 | 76.17±19.40 | 110.90±40.42 | 117.50±38.83 | 112.40±67.82 | 110.10±26.41 |
| CHO (mg/dL) | 95.87±13.22*** | 173.30±28.49 | 145.30±15.66* | 165.50±24.94 | 144.10±40.29* | 148.60±17.87 | 153.50±15.94 |
| GLU (mg/ dL) | 126.90±21.74*** | 209.00±51.98 | 203.60±25.81*** | 88.31±13.10*** | 77.43±8.60*** | 98.63±20.77*** | 76.88±5.69*** |
| TG (mg/dL) | 54.04±8.67 | 72.39±15.08 | 78.12±15.01 | 82.14±14.57 | 96.92±29.61* | 89.54±24.16 | 113.20±23.47*** |
| HDL (mg/dL) | 75.41±11.11*** | 132.20±18.07 | 116.20±10.97 | 132.20±17.42 | 115.80±31.68 | 120.40±12.78 | 125.50±13.38 |
| LDL (mg/dL) | 12.64±1.48*** | 25.54±8.05 | 19.77±3.57 | 23.00±6.36 | 20.13±6.83 | 21.22±4.34 | 22.47±3.22 |

| | | | | | | | |
|---|---|---|---|---|---|---|---|
| **•** Mean±S.D • p<0.05, **p<0.01, **p<0.001 vs. Vehicle **•** One-way ANOVA Dunnett's test | | | | | | | |

As a result of measuring organ weights, as can be seen in Table 4, the liver weight of all administered groups except for the enavogliflozin 10 mg/kg administration group was significantly less than that of the vehicle group, and an effect according to the administered group was not observed for the kidney weight.

**[Table 4]**

| **Necropsy (Fasting)** | **Lean** | **Veh** | **Lira** | **Enavogliflozin 3 mg/kg** | **Enavogliflozin 10 mg/kg** | **Enavogliflozin 3 mg/kg** | **Enavogliflozin 10 mg/kg** |
|---|---|---|---|---|---|---|---|
| | | | | | | **+ Lira** | |
| **Liver weight (g)** | **1.00±0.14*** | **1.25±0.28** | **1.04±0.18*** | **1.06±0.11*** | **1.10±0.20** | **0.98±0.10**** | **0.99±0.11**** |
| **Kidney weight (g)** | 0.32±0.04 | 0.37±0.03 | 0.36±0.05 | 0.37±0.03 | 0.40±0.06 | 0.38±0.04 | 0.37±0.03 |

| | | | | | | | |
|---|---|---|---|---|---|---|---|
| • Mean±S.D • *p<0.05, **p<0.01. ***p<0.001 vs. Vehicle • One-way ANOVA Dunnett's test | | | | | | | |

In summary, enavogliflozin exhibits a significant body weight reduction efficacy compared to the vehicle group, even when administered in combination with liraglutide at 10 nmol/kg, which is less than the commercial dose of liraglutide (100 nmol/kg/day = 3 mg/day in humans). Therefore, it is expected that the side effects of liraglutide can be reduced by achieving an excellent body weight reduction efficacy while reducing the dose of liraglutide.

Accordingly, the present inventors conceived the preparation of a combination formulation capable of increasing the medication convenience of patients while administering enavogliflozin in combination with reducing the dose of liraglutide, and prepared a depot composition comprising enavogliflozin and liraglutide as follows to evaluate its anti-obesity efficacy.

### [Preparation Examples 1 and 2]

As a depot composition comprising enavogliflozin and liraglutide, the depot compositions of Preparation Examples 1 and 2 were obtained according to the composition and manufacturing method of Table 5.

**[Table 5]**

| Prepar ation Examp le | Prepar ation Metho d | Poly mer Con cent ratio n / Typ e | Ad diti ve & Sp eci al Fe atu res | EN AV O Con cent ratio n (w/ w%) | LIR A Con cent ratio n (w/ w%) | Co ntin uou s Pha se( CP) Co ndit ion | 1 st Hom ogeni zatio n Cond ition (RP M) | 2nd Hom ogeni zatio n Cond ition (RP M) | CP Inje ctio n Spe ed / Inje ctio n Te mp erat ure (°C ) | Disc onti nuo us Phas e (DP) Inje ctio n Spee d | Volatil ization Condit ion Tempe rature / Time / RPM | Size D10/D 50/D9 0/D[4, 3] |
|---|---|---|---|---|---|---|---|---|---|---|---|---|
| **1** | W/O/ W | 35% PLG A RG 752 H | Na Ac 1% | 16.6 7 | 27.1 7 | 0.2 5% PV A 3L + 1% ma nnit ol | 4,000 RPM 3 min | 4,000 RPM 3 min | 1.5 L/m in 20 ° C | 5ml/ min | 25 °C / 3 hours / 150 | 3.4/26. 8/65.1/ 30.9 |
| **2** | W/O/ W | 30% PLA R20 2H | M g( O H) 2 10 % | 16.6 7 | 27.1 7 | 0.5 0% PV A 5L + 1% ma nnit ol | 4,000 RPM 3 min | 4,000 RPM 3 min | 1.5 L/m in 20 ° C | 5ml/ min | 30°C / 3 hours / 150 | 3.8/23. 1/63.7/ 29.1 |

Liraglutide was sufficiently dissolved in primary distilled water for 30 minutes or more to prepare a first aqueous phase solution (W). Enavogliflozin was used after being sufficiently dissolved in methanol for 30 minutes or more. The biodegradable polymer was dissolved in methylene chloride, which is an oil phase solvent. An oil phase solution (O) was prepared by mixing the solution in which enavogliflozin was dissolved in methanol and the solution in which the biodegradable polymer was dissolved in methylene chloride. The first aqueous phase solution (W) and the oil phase solution (O) were mixed and homogenized using a homogenizer to prepare a W/O emulsion. In addition, an aqueous solution containing polyvinyl alcohol (PVA) as a surfactant was prepared as a second aqueous phase solution.

When the W/O emulsion is injected through a rubber pipe utilizing a micro-liquid medicine injector, microparticles are generated while being exposed to the surfactant aqueous phase solution and simultaneously mixed by a Homo-Mixer. The organic solvent present in the microparticles is removed by rotating the mixed solution of the W/O emulsion and the surfactant aqueous phase solution at a constant speed. In order to remove the organic solvent, the preparation vessel temperature was adjusted to 15 to 35°C, and maintained for 3 hours. When the removal of the organic solvent was completed, the suspension was repeatedly washed with primary distilled water to remove residual polyvinyl alcohol, and the recovered microparticles were freeze-dried.

### [Experimental Example 2] Evaluation of anti-obesity effectiveness of Preparation Examples 1 and 2

For the evaluation of the anti-obesity effectiveness of the depot compositions of Preparation Examples 1 and 2, obesity was induced in 5-week-old male SD rats to be used as an animal model.

Obesity was induced by feeding a high-fat diet to rats for 13 weeks. To minimize the stress received by the experimental animals due to subcutaneous administration of the test substance, physiological saline was subcutaneously administered to all animals from 2 weeks before the administration of the test substance to perform pre-adaptation training for subcutaneous administration.

The test groups were set as a vehicle group (G1), an enavogliflozin single administration group (G2), a Saxenda^{™} (high-dose liraglutide) single administration group (G3), and groups administered every 2 or 4 weeks with a depot formulation in which enavogliflozin and low-dose liraglutide were encapsulated (G4 to G6) as follows (n=8 per each group). All test groups were administered with the test substance for 4 weeks through subcutaneous injection (S.C) of a 2 mg/kg volume including the test substance and vehicle.

**[Table 6]**

| Gro up | Group Name | Test Substance and Single Dose | Dosing Regimen |
|---|---|---|---|
| G1 | Vehicle | Vehicle (PBS) | Once a day |
| G2 | Enavogliflozin | Enavogliflozin (0.30 mg/kg) | Once a day |
| G3 | Saxenda^{™} | Liraglutide (0.31 mg/kg) | Once a day |
| G4 | Preparation Example 1(2W) | ENAVO (5.32mg/kg), LIRA(2.17mg/kg) | once every 2 weeks |
| G5 | Preparation Example 2(2W) | ENAVO (4.20mg/kg), LIRA(2.17mg/kg) | once every 2 weeks |
| G6 | Preparation Example 2(4W) | ENAVO (8.40mg/kg), LIRA(4.34mg/kg) | once every 4 weeks |

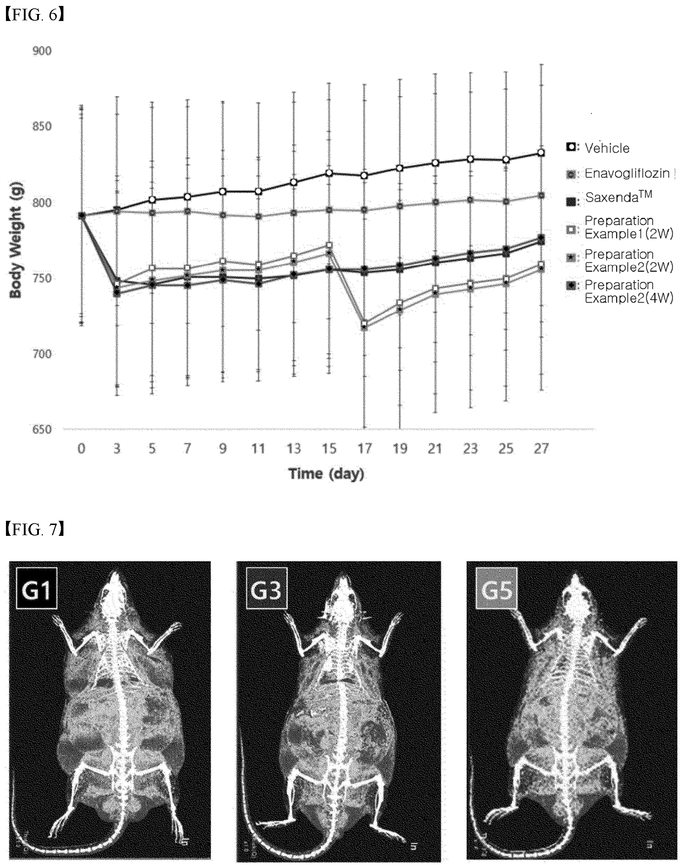

As a result of analyzing the body weight reduction rate for each group, as can be seen in Fig. 6, the body weight reduction effect of G2 compared to G1 was minimal (-3.39%), but the case of G4 (-8.85%) and G5 (-9.26%), which are the combination depot injection groups combined with low-dose liraglutide administered at 2-week intervals, showed superior body weight reduction effect than G3 (-7.09%), which is the high-dose liraglutide single administration group. In addition, the case of G6 (-6.75%), which is the combination depot injection group combined with low-dose liraglutide administered at 4-week intervals, showed a body weight reduction effect similar to G3 (-7.09%), which is the high-dose liraglutide single administration group. In the case of G6, compared to G3, which must be administered 28 times over 4 weeks, it is a formulation in which patient medication convenience is significantly improved in that it is a body weight reduction effect obtained through only a single injection administration.

Furthermore, as a result of analyzing the fat weight and the proportion of fat within tissues through body composition analysis, as presented in Table 7 and Fig. 7, the depot injection administration group G5 comprising enavogliflozin and low-dose liraglutide exhibited a reduction in fat mass similar to G3, which is the high-dose liraglutide single administration group, compared to the vehicle group (the red areas in Fig. 7 represent fat).

**[Table 7]**

| Body fat mass and muscle mass in SD rats fed 60% HFD during the experimental period while receiving test substances | | | | | | | | | | |
|---|---|---|---|---|---|---|---|---|---|---|
| Group | | | Value (g) | | | | BMD (g/cm ²) | Bone Area (cm²) | Bone Volu me (cm³) | FAT In Tissue (%) |
| | | | Total Mass | BMC | FAT | LEAN | | | | |
| G1 | N | Mea n | 856.16 | 19.457 4 | 358.86 84 | 477.83 79 | 0.244 6 | 79.56 94 | 11.76 86 | 42.689 3 |
| Vehicle | 8 | SD | 65.28 | 1.0066 | 58.857 0 | 28.749 3 | 0.009 5 | 3.468 3 | 0.608 8 | 4.3588 |
| G2 | N | Mea n | 820.03 | 18.827 7 | 311.12 94 | 490.07 05 | 0.242 3 | 77.72 63 | 11.38 77 | 38.628 1 |
| Enavogli flozin | 8 | SD | 73.05 | 1.0777 | 52.643 7 | 30.365 2 | 0.005 9 | 4.456 5 | 0.651 8 | 3.6568 |
| G3 | N | Mea n | 793.24 | 19.191 4 | 293.35 49 | 480.69 09 | 0.242 5 | 79.07 54 | 11.60 77 | 37.706 6 |
| Saxenda TM | 8 | SD | 65.20 | 1.6238 | 47.818 8 | 26.415 9 | 0.009 2 | 4.802 4 | 0.982 1 | 3.5796 |
| G4 | N | Mea n | 778.91 | 18.547 7 | 304.13 75 | 456.22 13 | 0.235 0 | 78.94 68 | 11.21 84 | 39.768 0 |
| Preparati on Example 1(2W) | 7 | SD | 79.42 | 1.4308 | 54.677 3 | 32.010 8 | 0.007 4 | 5.732 2 | 0.865 4 | 3.4135 |
| G5 | N | Mea n | 775.81 | 18.681 0 | 289.30 78 | 467.82 31 | 0.239 1 | 78.11 44 | 11.29 90 | 37.937 6 |
| Preparati on Example 2(2W) | 8 | SD | 72.93 | 0.9111 | 54.330 8 | 29.176 0 | 0.004 6 | 3.048 9 | 0.551 0 | 4.2511 |
| G6 | N | Mea n | 794.12 | 18.479 9 | 297.57 11 | 478.07 34 | 0.234 0* | 79.05 88 | 11.17 74 | 38.196 4 |
| Preparati on Example 2(4W) | 8 | SD | 55.28 | 0.5802 | 46.407 4 | 26.708 8 | 0.009 5 | 3.445 3 | 0.350 9 | 4.0630 |

| | | | | | | | | | | |
|---|---|---|---|---|---|---|---|---|---|---|
| BMC: bone mineral content; BMD: bone mineral density. Significant difference from G1 HFD vehicle control group by One-way ANOVA followed by the Dunnett's test: * p<0.05, ** p<0.01. | | | | | | | | | | |

In addition, as a result of serological analysis, as can be seen in Table 8, the LDL-C reduction result was significantly reduced in G4, which is the combination depot injection group comprising enavogliflozin and low-dose liraglutide, compared to the result of G3, which is the high-dose liraglutide single administration group. In addition, in G5 and G6, which are the combination depot injection groups comprising enavogliflozin and low-dose liraglutide, the TG level was also significantly reduced compared to the G1 group.

Furthermore, in the blood chemistry analysis, as shown in Table 8, the reduction in LDL-C was significantly greater in the combination depot group G4 comprising enavogliflozin and low-dose liraglutide compared to the result of the high-dose liraglutide single administration group G3. Moreover, in the combination depot groups G5 and G6 comprising enavogliflozin and low-dose liraglutide, TG levels were also significantly reduced compared to the G1 group.

**[Table 8] Blood chemistry analysis results of SD rats fed 60% HFD during the experimental period while receiving test substances**

| Group | | | Blood chemistry analysis | | | | | |
|---|---|---|---|---|---|---|---|---|
| | | | AST (U/L) | ALT (U/L) | TG (mg/dL) | T-Chol (mg/dL) | LDL-C (mg/dL) | HDL-C (mg/dL) |
| G1 | N | Mean | 339.63 | 198.13 | 79.25 | 96.75 | 6.88 | 22.63 |
| Vehicle | 8 | SD | 306.04 | 180.52 | 21.83 | 21.17 | 1.55 | 4.84 |
| G2 | N | Mean | 226.50 | 123.38 | 61.13 | 86.38 | 5.13 | 24.00 |
| Enavogliflozin | 8 | SD | 151.19 | 96.09 | 18.70 | 18.57 | 0.64 | 3.25 |
| G3 | N | Mean | 184.25 | 105.50 | 40.88** | 80.50 | 6.13 | 22.88 |
| Saxenda^{®} | 8 | SD | 84.12 | 99.47 | 10.26 | 15.72 | 0.99 | 4.64 |
| G4 | N | Mean | 167.29 | 72.86 | 52.71 | 78.86 | 4.71* | 22.57 |
| Preparation Example 1(2W ) | 7 | SD | 57.83 | 38.37 | 12.20 | 24.41 | 0.95 | 6.05 |
| G5 | N | Mea n | 157.75 | 73.63 | 49.63* | 74.38 | 5.00 | 22.25 |
| Prepa ration Exam ple 2(2W ) | 8 | SD | 79.28 | 43.23 | 10.93 | 16.07 | 1.41 | 3.96 |
| G6 | N | Mea n | 135.88 | 55.75 | 51.50* | 90.75 | 6.75 | 21.88 |
| Prepa ration Exam ple 2(4W ) | 8 | SD | 42.83 | 27.14 | 3.89 | 12.16 | 1.83 | 2.17 |

| | | | | | | | | |
|---|---|---|---|---|---|---|---|---|
| Significant difference from G1 vehicle control group by One-way ANOVA followed by the Dunnett's test: * p<0.05, ** p<0.01. AST: aspartate aminotransferase, ALT: alanine aminotransferase, TG: triglyceride T-Chol: total cholesterol, LDL-C: low density lipoprotein-cholesterol, HDL-C: high density lipoprotein-cholesterol. | | | | | | | | |

Furthermore, as shown in Table 9, which compares the internal organ weights of SD rats at necropsy after the end of the study, the absolute liver weight was significantly reduced in the combination depot groups G4, G5, and G6 comprising enavogliflozin and low-dose liraglutide compared to the result of the high-dose liraglutide single administration group G3. In addition, in the combination depot group G6, the relative organ weight of the liver was also significantly reduced compared to the G1 group.

**[Table 9]**

| Absolute organ weight (g) and relative organ weight (organ weight/body weight ratio) at sacrifice | | | | | | | |
|---|---|---|---|---|---|---|---|
| | | | | | Body | Absolute organ | Relative organ |
| Group | | | weight (g) | weight (g) | | weight | |
| | | | | liver | epididymal fat | liver | epididymal fat |
| G1 | N | Mean | 811.13 | 19.5015 | 26.1435 | 0.0240 | 0.0321 |
| Vehicle | 8 | SD | 60.05 | 3.0972 | 4.4436 | 0.0030 | 0.0044 |
| G2 | N | Mean | 777.17 | 18.6817 | 23.2977 | 0.0241 | 0.0295 |
| Enavogliflozin | 8 | SD | 71.98 | 2.8227 | 7.0975 | 0.0032 | 0.0065 |
| G3 | N | Mean | 744.09 | 17.1632 | 21.5237 | 0.0231 | 0.0287 |
| Saxenda^{®} | 8 | SD | 62.64 | 2.3700 | 4.5815 | 0.0027 | 0.0041 |
| G4 | N | Mean | 729.42 | 15.7746* | 21.6260 | 0.0218 | 0.0296 |
| Preparation Example 1(2W) | 7 | SD | 75.52 | 1.4751 | 3.9017 | 0.0025 | 0.0042 |
| G5 | N | Me an | 735.46 | 15.2485* | 20.6418 | 0.0206 | 0.0281 |
| Preparation Example 2(2W) | 8 | SD | 69.09 | 2.7544 | 3.2107 | 0.0024 | 0.0041 |
| G6 | N | Me an | 751.34 | 14.5738* * | 23.4828 | 0.0194** | 0.0311 |
| Preparation Example 2(4W) | 8 | SD | 52.72 | 1.1108 | 6.1003 | 0.0013 | 0.0071 |

| | | | | | | | |
|---|---|---|---|---|---|---|---|
| Significant difference from G1 HFD vehicle control group by One-way ANOVA followed by the Dunnett's test: * p<0.05, ** p<0.01. | | | | | | | |

In conclusion, in a rat model of obesity induced by 13 weeks of high-fat diet feeding, the combination depot formulations G4 and G5 comprising enavogliflozin and low-dose liraglutide administered once every 2 weeks demonstrated superior body weight reduction effects compared to the existing obesity treatment Saxenda^{™} (high-dose liraglutide administration) group G3. The 4-week interval combination depot G6 also showed a body weight reduction effect similar to G3. Furthermore, comprehensive analysis of fat weight, blood chemistry analysis, and absolute/relative liver weight results confirmed that the combination depot administration comprising enavogliflozin and low-dose liraglutide provides additional benefits in improving obesity-related metabolism that cannot be confirmed with high-dose liraglutide alone, while simultaneously reducing side effects, achieving comparable body weight loss, and furthermore, improving patient medication convenience.

Accordingly, further studies related to the sustained drug release of the depot composition were continued in the subsequent research.

### [Preparation Examples 3 to 10]

In order to secure sustained drug release of enavogliflozin and liraglutide, depot compositions comprising enavogliflozin and liraglutide were prepared through various attempts (S/O/W preparation method, spray drying preparation method, and a post-mixing preparation method where enavogliflozin and liraglutide were separately encapsulated into microparticles, recovered by freeze-drying, and then mixed). The specific manufacturing method is as follows.

In Preparation Examples 3 to 10, enavogliflozin and liraglutide acetate were used as active ingredients. They were manufactured using the S/O/W manufacturing method, the Spray drying manufacturing method, or a method of separately encapsulating each active ingredient in microparticles and then post-mixing them.

First, to prepare liraglutide acetate utilizing hydrophobic ion pairing, 10 g of liraglutide and 4.2 g of sodium acetate were dissolved in 400 ml of primary distilled water and mixed for 30 minutes. To the mixture, 10 ml of acetic acid (Glacial, Sigma) or 10 ml of 1N HCl was added to lower the pH, thereby precipitating liraglutide acetate, and the mixture was allowed to further react for 4 hours. Thereafter, it was repeatedly washed with primary distilled water and methanol, filtered through a 635 Mesh sieve, and the recovered liraglutide acetate was recovered by freeze-drying.

In the case of Preparation Example 3, a PLA polymer of R202H was dissolved in methylene chloride at a weight ratio of 30 w/w% and used, and enavogliflozin was completely dissolved in methanol at a weight ratio of 16.7 w/w%. Then, the polymer solution and solid-phase liraglutide acetate were mixed using a Homo-mixer at 10,000 RPM for 3 minutes to prepare a first mixed solution (S/O emulsion). Thereafter, the first mixed solution (S/O emulsion) was introduced into a 5L water bath containing a surfactant (PVA) at a weight ratio of 0.5%, and simultaneously a Homo-mixer was applied at 4,500 RPM for 3 minutes, thereby preparing a second mixed solution (S/O/W microparticles). For the solvent evaporation process, the water bath temperature was adjusted to 30°C, and the mixed solution was stirred at 150 RPM using an overhead stirrer for 3 hours. Thereafter, microparticles were recovered through centrifugation at 1,500 to 3,000 RPM for 3 minutes, and washed 3 times repeatedly using primary distilled water to wash away unencapsulated active ingredients and the surfactant. Thereafter, freeze-drying was performed at - 40°C to 5°C for 48 hours, thereby recovering microparticle powder. The recovered microparticles were passed through a 200 Mesh sieve, thereby finally securing the microparticles.

In the case of Preparation Example 4, a PLA polymer of R203H was dissolved in methylene chloride at a weight ratio of 23 w/w% and used, and enavogliflozin was completely dissolved in methanol at a weight ratio of 16.7 w/w%. Then, the polymer solution and solid-phase liraglutide acetate were mixed using a Homo-mixer at 10,000 RPM for 3 minutes to prepare a first mixed solution (S/O emulsion). Thereafter, the first mixed solution (S/O emulsion) was introduced into a 1L water bath containing a surfactant (PVA, Gohsenol EG-40PW) at a weight ratio of 1.0 w/w% while applying a Homo-mixer at 3,500 RPM for 3 minutes, thereby preparing a second mixed solution (S/O/W microparticles). For the solvent evaporation process, the water bath temperature was adjusted to 30°C, and the solvent evaporation process was carried out at 150 RPM using an overhead stirrer for 3 hours. Thereafter, microparticles were recovered through centrifugation at 1,500 to 3,000 RPM for 3 minutes, and washed 3 times repeatedly using primary distilled water to wash away unencapsulated active ingredients and the surfactant. Thereafter, freeze-drying was performed at -40°C to 5°C for 48 hours, thereby recovering microparticle powder. The recovered microparticles were passed through a 200 Mesh sieve, thereby finally securing the microparticles.

In the case of Preparation Example 5, a PLA polymer of R203H was dissolved in methylene chloride at a weight ratio of 15 w/w% and used, and enavogliflozin was completely dissolved in methanol at a weight ratio of 16.7 w/w%. Then, the polymer solution and solid-phase liraglutide acetate were mixed using a Homo-mixer at 15,000 RPM for 3 minutes to make a mixed solution (S/O emulsion), and then microparticles were prepared using a Spray dryer (BUCHI, Mini B-290). Here, the mixed solution was injected at a speed of 1.0 ml/min using an injection pump, the Inlet temperature was set to 55 to 75°C, the Outlet temperature was set to 36 to 42°C, and the mixed solution was atomized at a spray pressure of 20 to 50 psi, thereby manufacturing and recovering microparticles.

In the case of Preparation Example 6, a PLA polymer of R203H was dissolved in methylene chloride at a weight ratio of 23 w/w% and used, and enavogliflozin was completely dissolved in methanol at a weight ratio of 16.7 w/w%. Then, the polymer solution and solid-phase liraglutide acetate were mixed using a Homo-mixer at 10,000 RPM for 3 minutes to prepare a first mixed solution (S/O emulsion). Thereafter, the first mixed solution (S/O emulsion) was introduced into a 1L water bath containing a surfactant (PVA, Merck 05P) at a weight ratio of 1.0%, and simultaneously a Homo-mixer was applied at 3,500 RPM for 3 minutes, thereby preparing a second mixed solution (S/O/W microparticles). For the solvent evaporation process, the water bath temperature was adjusted to 35°C, and a solvent evaporation process was performed at 150 RPM using an overhead stirrer for 3 hours. Thereafter, microparticles were recovered through centrifugation at 1,500 to 3,000 RPM for 3 minutes, and washed 3 times repeatedly using primary distilled water to wash away unencapsulated active ingredients and the surfactant. Thereafter, freeze-drying was performed at -40°C to 5°C for 48 hours, thereby recovering microparticle powder. The recovered microparticles were passed through a 200 Mesh sieve, thereby finally securing the microparticles.

In the case of Preparation Example 7, enavogliflozin and liraglutide acetate were separately manufactured without being mixed. For manufacturing enavogliflozin microparticles, a PLA polymer of R203H was dissolved in methylene chloride at a weight ratio of 23 w/w% and used, and after completely dissolving enavogliflozin in methanol at a weight ratio of 16.7 w/w%, the polymer solution was mixed using a Homo-mixer at 10,000 RPM for 3 minutes to prepare a first mixed solution (S/O emulsion). Thereafter, the first mixed solution (S/O emulsion) was introduced into a 1L water bath containing a surfactant (PVA, Merck 05P) at a weight ratio of 1.0 w/w% while applying a Homo-mixer at 3,500 RPM for 3 minutes, thereby preparing a second mixed solution (S/O/W microparticles). For the solvent evaporation process, the water bath temperature was adjusted to 30°C, and the solvent evaporation process was carried out at 150 RPM using an overhead stirrer for 3 hours. Thereafter, microparticles were recovered through centrifugation at 1,500 to 3,000 RPM for 3 minutes, and washed 3 times repeatedly using primary distilled water to wash away unencapsulated active ingredients and the surfactant. Thereafter, freeze-drying was performed at -40°C to 5°C for 48 hours to recover microparticle powder. The recovered microparticles were passed through a 200 Mesh sieve, thereby finally securing the microparticles. In addition, for manufacturing liraglutide acetate microparticles, a PLA polymer of R203H was dissolved in methylene chloride at a weight ratio of 23 w/w% and used, and other manufacturing processes were carried out identically to the microparticle manufacturing conditions in which enavogliflozin was separately encapsulated as described above.

In the case of Preparation Example 8, a PLA polymer of R202H was dissolved in methylene chloride at a weight ratio of 30 w/w% and used, and after completely dissolving enavogliflozin in methanol at a weight ratio of 16.7 w/w%, the polymer solution and solid-phase liraglutide acetate were mixed using a Homo-mixer at 15,000 RPM for 3 minutes to prepare a first mixed solution (S/O emulsion).

Thereafter, the first mixed solution (S/O emulsion) was introduced into a 1L water bath containing a surfactant (PVA, Merck 05P) at a weight ratio of 1.0 w/w% while applying a Homo-mixer at 3,500 RPM for 3 minutes, thereby preparing a second mixed solution (S/O/W microparticles). For the solvent evaporation process, the water bath temperature was adjusted to 30°C, and a solvent evaporation process was performed at 150 RPM using an overhead stirrer for 3 hours. Thereafter, microparticles were recovered through centrifugation at 1,500 to 3,000 RPM for 3 minutes, and the recovered microparticles were washed once utilizing PBST (Tween 80, 0.05%) and then washed 3 times repeatedly utilizing primary distilled water to wash away unencapsulated active ingredients and the surfactant. Thereafter, freeze-drying was performed at - 40°C to 5°C for 48 hours, thereby recovering microparticle powder. The recovered microparticles were passed through a 200 Mesh sieve, thereby finally securing the microparticles.

In the case of Preparation Example 9, a PLA polymer of R203H was dissolved in methylene chloride at a weight ratio of 23 w/w% and used. After completely dissolving enavogliflozin in methanol at a weight ratio of 16.7 w/w%, 1N HCl instead of acetic acid was added to the mixture to lower the pH, thereby precipitating liraglutide acetate. Then, the polymer solution and solid-phase liraglutide acetate were mixed using a Homo-mixer at 15,000 RPM for 2.5 minutes to prepare a first mixed solution (S/O emulsion). Thereafter, the first mixed solution (S/O emulsion) was introduced into a 1L water bath containing a surfactant (PVA, Merck 05P) at a weight ratio of 1.0% while applying a Homo-mixer at 3,500 RPM for 3 minutes, thereby preparing a second mixed solution (S/O/W microparticles). For the solvent evaporation process, the water bath temperature was adjusted to 30°C, and the solvent evaporation process was carried out at 150 RPM using an overhead stirrer for 3 hours. Thereafter, microparticles were recovered through centrifugation at 1,500 to 3,000 RPM for 3 minutes, and the recovered microparticles were washed once utilizing PBST (Tween 80, 0.05%) and then washed 3 times repeatedly utilizing primary distilled water to wash away unencapsulated active ingredients and the surfactant. Thereafter, freeze-drying was performed at -40°C to 5°C for 48 hours to recover microparticle powder. The recovered microparticles were passed through a 200 Mesh sieve, thereby finally securing the microparticles.

In the case of Preparation Example 10, a PLA polymer of R203H was dissolved in methylene chloride at a weight ratio of 23 w/w% and used. After completely dissolving enavogliflozin in methanol at a weight ratio of 16.7 w/w%, the polymer solution and solid-phase liraglutide acetate were mixed.

In the case of Preparation Example 10, a PLA polymer of R203H was dissolved in methylene chloride at a weight ratio of 23% and used. After completely dissolving enavogliflozin in methanol at a weight ratio of 16.7%, the polymer solution and the solid-phase liraglutide acetate-which was secured through a recovery process after precipitation by repeatedly washing with primary distilled water and completing the final washing utilizing methanol-were mixed. Then, the mixture was mixed using a Homo-mixer at 10,000 RPM for 3 minutes to prepare a first mixed solution (S/O emulsion). Thereafter, the first mixed solution (S/O emulsion) was introduced into a 1L water bath containing a surfactant (PVA, Merck 05P) at a weight ratio of 1.0%, and simultaneously a Homo-mixer was applied at 3,500 RPM for 3 minutes to prepare a second mixed solution (S/O/W microparticles). For the solvent evaporation process, the water bath temperature was adjusted to 30°C, and the mixed solution was stirred at 150 RPM using an overhead stirrer for 3 hours. Thereafter, microparticles were recovered through centrifugation at 1,500 to 3,000 RPM for 3 minutes, and washed once utilizing PBST (Tween 80, 0.05%) and then washed 3 times repeatedly utilizing primary distilled water to wash away unencapsulated active ingredients and the surfactant. Thereafter, freeze-drying was performed at -40°C to 5°C for 48 hours to recover microparticle powder. The recovered microparticles were passed through a 200 Mesh sieve, thereby finally securing the microparticles.

In order to check the diameter and particle size distribution of the combination microparticles containing enavogliflozin and liraglutide acetate, analysis was performed using a Malvern particle size analyzer.

Specifically, the depot compositions of Preparation Examples 3 to 10 were obtained according to the composition and manufacturing method of Table 10.

**[Table 10]**

| **Preparation Example** | **Preparation Method** | **Polymer Concentration & Type** | **Additive & Special Features** | **ENAVO Concentration w/w %** | **Continuous Phase Condition** | **1st Homogenization Condition (RPM)** | **2nd Homogenization Condition (RPM)** | **Solvent Evaporation Condition (Temp/ Time/R PM)** | **Particle Size D10/D50/D 90/D[4,3]** |
|---|---|---|---|---|---|---|---|---|---|
| **3** | S/O/W | 30% PL A R20 2H | LIRA Acetate | 16.7 | 0.50% PVA 5L Gohsenol EG-40PW | 10,0 00 3 min | 4,50 0 3 min | 30°C / 3 / 150 | 2.3/13.0/37. 4/1**6.9** |
| **4** | S/O/W | 23% PL A R20 3H | LIRA Acetate | 16.7 | 1.0%, PVA 1L Gohsenol EG-40PW | 10,0 00 3 min | 3,50 0 3 min | 30°C / 3 / 150 | 1.9/13.0/34. 2/**15.7** |
| **5** | Spray Drying | 15% PL A R20 3H | LIRA Acetate | 16.7 | 1.0%, PVA 1L Merck PVA 05P | 15,0 00 3 min | N.A | N.A | 2.7/17.6/44. 2/**21.6** |
| **6** | S/O/W | 23% PL A R20 3H | LIRA Acetate | 16.7 | 1.0%, PVA 1L Merck PVA 05P | 10,0 00 3 min | 3,50 0 3 min | 35°C / 3 / 150 | 2.0/15.6/36. 4/**17.5** |
| **7** | O/W | 23% PL A R20 2H | ENA VO separately prepared | 16.7 | 1.0%, PVA 1L Merck PVA 05P | 10,0 00 3 min | 3,50 0 3 min | 30°C / 3 / 150 | 2.0/14.6/33. 3/**18.1** |
| | S/O/W | 23% PL A R20 3H | LIRA Acetate | N.A | 1.0%, PVA 1L Merck PVA 05P | 10,0 00 3 min | 3,50 0 3 min | 30°C / 3 / 150 | 2.0/16.6/37. 6/**22.2** |
| **8** | S/O/W | 30% PL A R20 2H | LIRA - Aceta te /PBS T 0.05 % washi ng | 16.7 | 1.0%, PVA 1L Merck PVA 05P | 15,0 00 3 min | 3,50 0 3 min | 30°C / 3 / 150 | 8.0/31.2/64. 0/**34.0** |
| **9** | S/O/W | 23% PL A R20 3H | LIRA - Aceta te (HCl) | 16.7 | 1.0%, PVA 1L Merck PVA 05P | 15,0 00 2.5 min | 3,50 0 3 min | 30°C / 3 / 150 | 5.2/**2**4**.**4/51. 2/**26.9** |
| **10** | S/O/W | 30% PL A R20 2H | LIRA - Acetate/ MeOH Washing | 16.7 | 1.0%, PVA 1L Merck PVA 05P | 10,0 00 3 min | 3,50 0 3 min | 30°C / 3 / 150 | 7.4/28.8/65. 1/**32.1** |

### [Experimental Example 3] PK analysis according to administration of depot compositions of Preparation Examples 3 to 10

For PK analysis according to the administration of the depot compositions of Preparation Examples 3 to 10, 5-week-old male SD rats were used as an animal model.

As shown in Table 11, the test groups were set as an enavogliflozin + liraglutide combination administration group (G1) and the depot formulation administration groups of Preparation Examples 3 to 10 in which enavogliflozin and liraglutide were encapsulated (G2 to G9) as follows (G1: n=3, G2 to G9: n=2 per each group).

**[Table 11]**

| Test Substance | | Dosing Regimen | Dose (mg/kg) | Volume (mL/kg) |
|---|---|---|---|---|
| G1 | enavogliflozin + liraglutide | SC once daily | enavogliflozin 0.093+ liraglutide 0.186 | enavogliflozin 0.93+ liraglutide 1.86 |
| G2 | Preparation Example 3 | | enavogliflozin 1.87 + liraglutide 2.60 | |
| G3 | Preparation Example 4 | | enavogliflozin 2.86 + liraglutide 2.60 | |
| G4 | Preparation Example 5 | | enavogliflozin 2.77 + liraglutide 2.60 | |
| G5 | Preparation Example 6 | SC single administration (single dose) | enavogliflozin 3.00 + liraglutide 2.60 | 1.2 |
| G6 | Preparation Example 7 | | enavogliflozin 2.60 + liraglutide 2.60 | |
| G7 | Preparation Example 8 | | enavogliflozin 2.30 + liraglutide 2.60 | |
| G8 | Preparation Example 9 | | enavogliflozin 1.68 + liraglutide 2.60 | |
| G9 | Preparation Example 10 | | enavogliflozin 1.86 + liraglutide 2.60 | |

Plasma samples were collected at designated times, and samples were stored at -70°C until analyzed using an LC-MS/MS system. Protein precipitation was performed on 20 µL of plasma samples using 180 µL of acetonitrile containing an internal standard. After mixing and centrifugation, the supernatant (150 µL) was analyzed with an LC-MS/MS system equipped with an MRM mode. LC/MS/MS data were analyzed by Analyst 1.6.3. PK parameters were calculated by non-compartmental analysis using WinNonlin software.

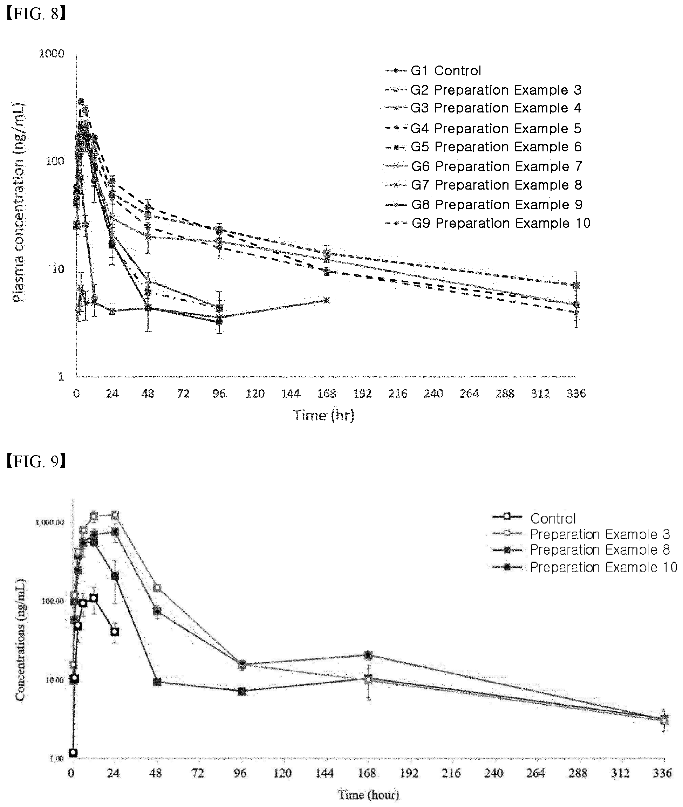

Fig. 8 shows the PK analysis results of enavogliflozin according to the administration of each test substance.

The sustained drug release level of the drug was evaluated through the plasma drug concentrations measured at 4 weeks (a total of 672 hours) after administration of the test substances. As a result, compared to the ENAVO control drug administration group (G1), the drug was detected at the 2-week time point (336 h) in groups G2, G4, G7, and G9. In addition, compared to the ENAVO control drug administration group (G1), the drug was detected up to the 4-week time point (672 h) in groups G7 and G9, confirming that the drug was continuously released.

Figure 9 shows the PK analysis results of liraglutide according to the administration of each test substance.

The sustained drug release level was evaluated through the plasma drug concentrations measured at 4 weeks (a total of 672 hours) after administration of the test substances. As a result, compared to the LIRA control group (G1), drug was detected at the 2-week time point (336 h) in groups G2, G7, and G9, confirming that sustained drug release was achieved.

In summary, in the case of the depot compositions of Preparation Example 3, Preparation Example 8, and Preparation Example 10, it was confirmed that both enavogliflozin and liraglutide exhibited sustained drug release for more than 2 weeks.

More preferably, it was confirmed that the liraglutide drug release from the depot composition of Preparation Example 10 was maintained at a relatively higher level at the 2-week time point (336 h) compared to the other Preparation Examples.

## Claims

1. A pharmaceutical composition for use in anti-obesity comprising enavogliflozin as an active ingredient, **characterized by** combination with a low dose of liraglutide.

2. The pharmaceutical composition for use in anti-obesity according to claim 1, wherein the low-dose of liraglutide is a dose of less than 3 mg/day of liraglutide.

3. The pharmaceutical composition for use in anti-obesity according to claim 1, wherein the dose of enavogliflozin is 0.3 to 1.5 mg/day and the dose of liraglutide is 1.2 to 2.4 mg/day.

4. The pharmaceutical composition for use in anti-obesity according to claim 1, wherein the dose of enavogliflozin is 0.6 to 1.2 mg/day and the dose of liraglutide is 1.5 to 2 mg/day.

5. The pharmaceutical composition for use in anti-obesity according to claim 1, wherein enavogliflozin is administered orally and liraglutide is administered by subcutaneous injection.
